# EUROPEAN PATENT SPECIFICATION

(11) **EP 0 562 029 B1**
(45) Date of publication and mention of the grant of the patent: **21.07.1999**
(21) Application number: 92903468.4
(22) Date of filing: 13.12.1991
(51) Int. Cl.: B67D 5/00, A61M 5/178

(54) **VARIABLE PROPORTION DISPENSER**
MEHRKOMPONENTEN SPENDER MIT VERWÄHLBAREM MISCHUNGSVERHÄLTNIS
DISTRIBUTEUR DE PROPORTIONS VARIABLES

(30) Priority: 14.12.1990 US 628271
(43) Date of publication of application: 29.09.1993
(73) Proprietor: Habley Medical Technology Corporation, Laguna Hills California 92653 (US)
(72) Inventor: SMEDLEY, William, H., Lake Elsinore, CA 92330 (US); FOSTER, Clark, B., Laguna Niguel, CA 92677 (US); HABER, Terry, M., Lake Forest, CA 92630 (US)
(74) Representative: Manitz, Finsterwald & Partner
(86) International application number: US9109379
(87) International publication number: WO9210425

(56) References cited:
- EP-A- 0 313 519
- DE-C- 137 660
- GB-A- 2 172 937
- US-A- 3 162 217
- US-A- 4 040 420
- US-A- 4 381 778
- US-A- 4 962 868

## Description

### BACKGROUND OF THE INVENTION

The present invention relates to a variable proportion dispenser of the kind as defined in the preamble of claim 1. A variable proportion dispenser of this kind is disclosed in EP-A-0 313 519.

Human insulin is of two basic types: regular and NPH. Insulin users use all regular insulin, all NPH or a mixture of the two insulins, typically 70% NPH and 30% regular. However, if one were to want a combination of regular and NPH other than the commercially available 70%/30% mixture, the user would need to have two sets of insulin injection syringes and would have to make two separate injections.

With the known variable proportion dispenser as disclosed in EP-A-0 313 519 each of the drive stems is formed by a toothed rack activated by an associated rack advance means. The rack advance means are activated by a common drive shaft. By choosing different rack advance means or by the use of toothed racks having different pitches the first and second movable elements can be driven with different feed.

### SUMMARY OF THE INVENTION

The variable proportion dispenser according to the present invention is characterized in that it further comprises:
Reciprocal drive means acting on said first and second drive stems for driving the first and second movable elements from the first and second starting positions towards the first and second ending positions in a cyclic manner;
said reciprocal drive means including:
first and second one-way drive devices for driving the first and second drive stems in a delivery direction during each cycle of said reciprocal drive means; each of said one-way drive devices comprising a reciprocating driver for driving the respective drive stem towards the respective container in the delivery direction and being movable relative to the drive stem in the reverse direction; and at least one of said one-way drive devices comprising means for adjusting the distance the reciprocating driver and the drive stem associated therewith travel in the delivery direction relative to the distance the reciprocating driver of the other one-way drive device and the other drive stem associated therewith travel in this delivery direction during each cycle of the reciprocal drive means.

Thus, the present invention is directed to a variable proportion dispenser, especially useful for dispensing different types of insulin in amounts and proportions selected by the user. Once the combined dosage is selected, both in amount and proportion, the same dosage will be automatically provided for each actuation cycle of the dispenser. The invention, as an insulin delivery system, permits the total amount of the insulin injected and the proportion NPH and regular human insulin to be user selected.

The variable proportion dispenser includes a housing which houses two or more variable volume containers, typically pharmaceutical cartridges. A reciprocating drive assembly is used to dispense predetermined amounts of the contents of the cartridges in predetermined proportions. The amounts and proportions, once set, remain the same for each actuation of the drive assembly. The reciprocating drive assembly includes a sliding body mounted to the housing. The sliding body moves between first and second axial positions during each cycle of the dispenser.

The drive assembly also includes a one-way drive device carried by the sliding body and a drive stem engaged by the one-way drive device during each delivery stroke. The one-way drive device preferably includes a threaded dosage adjuster which mounts within a threaded hole within the sliding body. The one way drive device also includes a reciprocating driver positioned between dosage adjuster and the cartridge. The dosage adjuster and reciprocating driver are axially aligned and configured so that when the sliding body, and dosage adjuster therewith, are driven during the delivery stroke from the first position towards the second position, that is towards the cartridge, the opposed ends of the dosage adjuster and reciprocating driver engage and the reciprocating driver is driven towards the cartridge. The reciprocating driver has a lower end which engages the drive stem during the delivery stroke to drive the drive stem against the piston in the cartridge. The lower end of the reciprocating driver and the drive stem are configured so the reciprocating drive ratchets back over the drive stem during the return stroke. Thus, the reciprocating driver acts as a one-way linear driver.

The reciprocating driver preferably has a collar positioned to engage a driver stop associated with the housing to limit the movement of the reciprocating driver on the return stroke away from the cartridge. Whether or not the collar contacts the driver stop is determined by the axial position of the dosage adjuster within the sliding body. For example, if the dosage adjuster is fully threaded into the sliding body, the collar will typically not contact the driver stop so that the adjacent ends of the dosage adjuster and reciprocating driver remain engaged throughout the cycle. However, if the dosage adjuster is moved away from the fully threaded position a sufficient amount, then the collar will contact the driver stop before the sliding body has reached its first position on the return stroke. This causes the opposed ends of the dosage adjuster and reciprocating driver to disengage. During the next delivery stroke, during which the sliding body is moved from the first position to the second position, the dosage adjuster does not contact the reciprocating driver for an initial portion of the stroke. This results in a decrease in the volume of the contents driven from the cartridge. Also, by individually adjusting the dosage adjusters, the point at which the dosage adjusters contact their respective reciprocating drivers can be changed. This permits the user to adjust the proportions and amounts of the components dispensed from the cartridges during each delivery stroke.

In a further advantageous embodiment of the invention a visual indicator is provided which permits the user to easily determine the amount of each component which is to be delivered before the delivery stroke. In one embodiment this is achieved using a visual dose indicator which moves axially according to the proposed dose. A separate dose indicator is used for each component.

Some users may suffer from a certain degree of confusion; the simultaneous visual display of two (or more) dose indicators when setting the dose for each component could create problems for these users. With a two-component dispenser, it is preferred that the dose indicator for each component be visually perceptible from opposite sides of the dispenser. Thus, when a user sets the dose, by rotating the dose adjustor, only one dose indicator is visible. This helps to ensure that the user does not become confused as to the dose selected.

In the present embodiments the axial position of the reciprocating driver prior to the delivery stroke determines the dose. The visual indication of this axial movement can be magnified by the dose indicator. For example, assume that an axial movement of three millimeters by the reciprocating driver corresponds to one unit of medication. With the present invention, the dose indicator can be driven in such a way that the dose indicator moves six millimeters for every three millimeters the reciprocating driver moves. This permits the units of medication markings, typically carried by the sliding body, to be spaced twice as far apart as would otherwise be possible thus greatly enhancing ease of use and accuracy. Of course, other ratios between the movement of the dose indicator and the movement of the reciprocating driver, and thus of the piston within the cartridge (either greater than one-to-one or less than one-to-one) could be used as well.

In one embodiment the amplification is achieved by providing two sets of threads on the threaded dose adjustor. For example, a right-hand set of threads can be used to drive the dose adjustor within the sliding body and a left-hand set of threads, also on the dose adjustor, can be used to drive the dose indicator. The right-hand threads on the dose adjustor, which engage right-hand threads within the sliding body, cause the dose adjustor to move one pitch length for each rotation of the dose adjustor. However, the dose indicator engages the left-hand threads of the dose adjustor and is prevented from rotating with the dose adjustor but is allowed to move axially. This causes the dose indicator to move one pitch length along the dose adjustor. Thus, the dose indicator moves within the sliding body two pitch lengths for each pitch length the dose adjustor moves. Accordingly, assuming the thread pitches of the left and right-hand threads are the same, the dose indicator moves twice the distance the dose adjustor moves within the sliding body. After the delivery stroke, once the user returns the sliding body back to its predelivery stroke, the dose adjustor and the dose indicator will return to the same positions within the sliding body as before the delivery stroke. Thus, if the user wishes to repeat a stroke with the same proportions, no adjustments need to be made.

One of the primary advantages of the invention is that it permits the user to adjust both the quantity and proportion of the two components to be delivered by the dispenser. The setting stays the same for multiple doses without the need for any additional adjustment.

Other features and advantages of the invention will appear from the following description in which the preferred embodiments have been set forth in detail in conjunction with the accompanying drawings.

### BRIEF DESCRIPTION OF THE DRAWINGS

Fig. 1 is an overall isometric view of a variable proportion dispenser made according to the invention with the sliding body in the first, pre-delivery position;
Fig. 2 is an exploded isometric view of the dispenser of Fig. 1;
Fig. 3 is a cross-sectional view of the dispenser of Fig. 1 with the cap removed, the sliding body in the first, predelivery position and one of the dosage adjusters repositioned within the sliding body;
Fig. 3A is an enlarged view of a portion of the dispenser of Fig. 3;
Fig. 4 shows the dispenser of Fig. 3 with the cap replaced and with the sliding body in the second, post-delivery position following dispensing of the two components in different proportions;
Fig. 5 shows the dispenser of Fig. 4 with the sliding body moved from the post-delivery position of Fig. 4 to an intermediate position;
Fig. 6 is an overall isometric view of an alternative embodiment of the variable proportion dispenser shown in Fig. 1 in the first, pre-delivery position with a dosing control knob pulled out away from the sliding body to facilitate adjustment;
Fig. 7 is an exploded isometric view of the dispenser of Fig. 6;
Fig. 8 is a cross-sectional view of the dispenser of Fig. 6. in the first, predelivery position;
Fig. 8A is an enlarged cross-sectional view of a portion of the dispenser of Fig. 8 with the right-hand dose adjustor axially pulled away from the sliding body and after having been rotated two complete revolutions so the dose indicator has moved within the sliding body a distance equal to four pitch lengths; and
Fig. 8B is an enlarged cross-sectional view of the lower portion of the dispenser of Fig. 8 illustrating, in somewhat exaggerated form, fluid flow from the right-hand cartridge, through the spike, past the check valve, through the common passageway and into the double-ended needle shown in phantom.

### DESCRIPTION OF THE PREFERRED EMBODIMENT

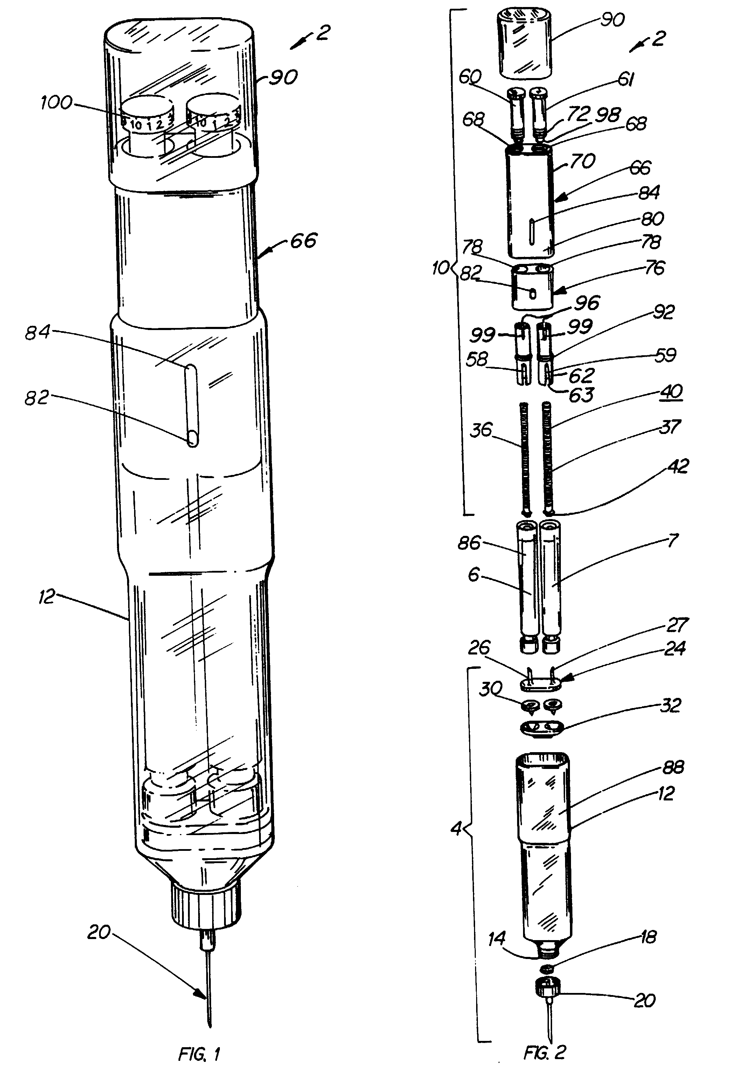

Fig. 1 illustrates a variable proportion dispenser 2 particularly suited for dispensing insulin. As shown in Fig. 2, dispenser 2 includes broadly a housing assembly 4, first and second insulin containing cartridges 6, 7 and a reciprocating drive assembly 10.

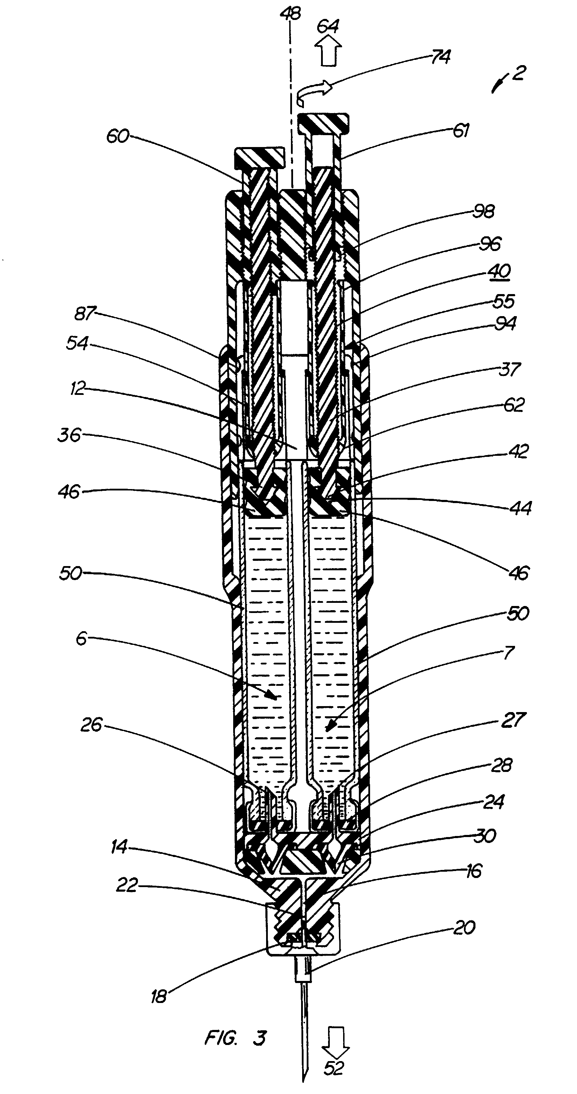

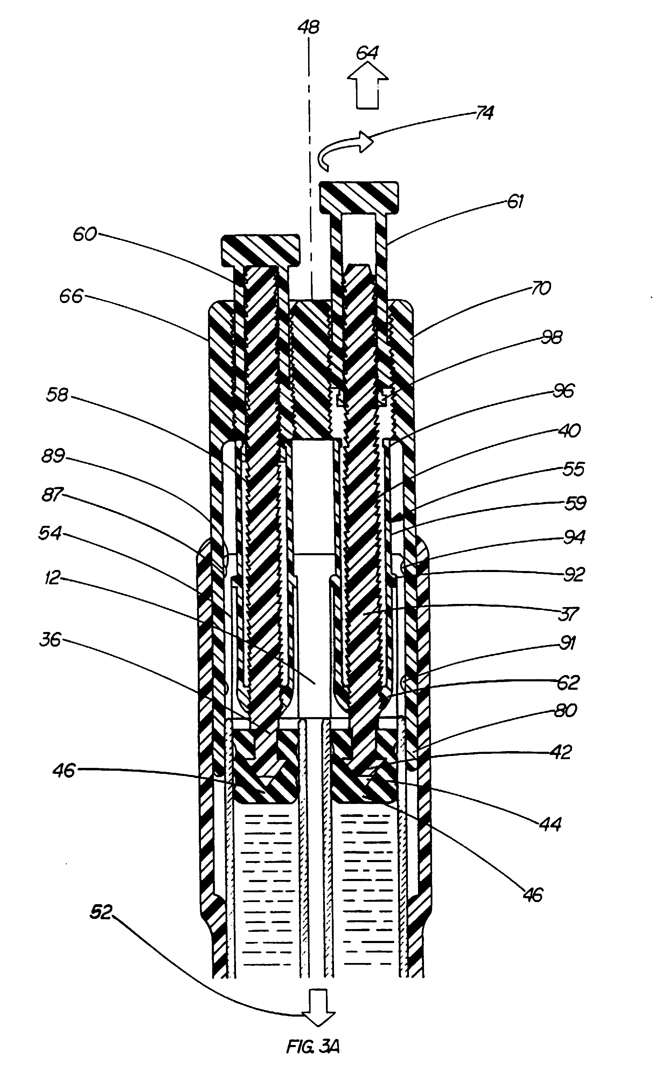

Turning now also to Fig. 3, housing assembly 4 is seen to include a housing 12, preferably made of a clear plastic material such as polycarbonate, so the user can see the contents of cartridges 6, 7. Housing 12 includes a threaded tip 14 having a central bore 16 formed therein. An elastomeric septum 18 is mounted to the end of tip 14. A double-ended needle assembly 20 has an inner end 22 which pierces septum 18 and is positioned within bore 16 to provide a conduit from bore 16 through needle assembly 20 during use. Needle assembly 20 is preferably replaced after each use.

Cartridges 6, 7 are housed within the interior of housing 12. A dual spike 24 having sharpened spike tips 26, 27 is used to pierce the septums 28 at the ends of cartridges 6, 7. A pair of elastomeric check valves 30 are positioned adjacent dual spike 24 by a check valve adapter 32. As shown in Fig. 3, this provides a pathway from the interiors of cartridges 6, 7, through spike tips 26, 27, past check valves 30, into bore 16 and through needle assembly 20. However, the capillary restrictions created within spike tips 26, 27 and needle assembly 20 and the restrictions provided by check valves 30 and septum 18 keep the contents of cartridges 6, 7 from leaking from dispenser 2.

Reciprocating drive assembly 10 includes first and second drive stems 36, 37 having serrated outer surfaces 40 and coned tips 42. Coned tips 42 are housed within complementary regions 44 formed within pistons 46 of cartridges 6, 7. Thus, movement of drive stems 36, 37 parallel to axis 48 will drive pistons 46 within the barrels 50 of cartridges 6, 7. Drive stems 36, 37 are driven in the direction of arrow 52 by one-way drive devices 54, 55. Drive devices 54, 55 include reciprocating drivers 58, 59 and dosage adjusters 60, 61. Devices 54, 55 are hollow to accommodate drive stems 36, 37. Drivers 58, 59 each include a stem engaging end 62 having serrations or teeth which complementarily engage the serrated outer surface 40 of its associated drive stem 36, 37. The serrations or teeth are configured such that movement of reciprocating drivers 58, 59 in the direction of arrow 52 causes stem engaging ends 62 to firmly grip the associated drive stems 36, 37, thus forcing piston 46 in the direction of arrow 52. However, movement in the direction of arrow 64, that is in the direction of the return stroke, allows stem engaging end 62, which has slits 63 which allow end 62 to dilate, to slide over serrated outer surface 40 so that the reciprocal movement of reciprocating drivers 58, 59 act in a ratcheting manner driving pistons 46 in the direction of arrow 52 but not in the reverse direction of arrow 64.

Reciprocating drive assembly 10 also includes a sliding body 66 having a pair of internally threaded holes 68 formed at one end 70 of sliding body 66. Dosage adjusters 60, 61 each include external threads 72 which engage threaded holes 68 to permit the user to adjust the axial positions of dosage adjusters 60, 61 relative to sliding body 66 as suggested in Fig. 3 by arrow 74.

Reciprocating drive assembly 10 also includes a limit guide 76 having parallel bores 78 through which reciprocating drivers 58, 59 and drive stems 36, 37 pass. The lower end 80 of sliding body 66 is hollow for receipt of limit guide 76. Limit guide 76 has an outwardly projecting rib 82, see Figs. 1 and 2, which rides within an axially extending slot 84 formed in end 80 of sliding body 66. Limit guide 76 is secured to housing 12 through the attachment of rib 82 to the inner wall of housing 12. The upper portion 88 of housing 12 is enlarged to accommodate sliding body 66. Movement of sliding body 66 and dosage adjusters 60, 61 is limited by engagement of rib 82 with the ends of slot 84. Sliding body 66 includes projections 87 which engage appropriately positioned indentations 89, 91 formed in limit guide 76 to act as detentes to help keep sliding body 66 in the pre-delivery and post-delivery positions.

Dispenser 2 is shown in Fig. 3 after a cap 90 has been removed, after sliding body has been moved from its second, post-delivery position of Fig. 1 to its first, predelivery position, and after dosage adjuster 61 has been adjusted by rotating in the direction of arrow 74. Doing so causes dosage adjuster 61 to separate from reciprocating driver 59 as shown in Fig. 3. This occurs because reciprocating driver has a collar 92 which engages an inwardly extending, annular driver stop 94 to prevent any further movement of reciprocating driver 59 in the direction of arrow 64. As seen in Fig. 3A, the ends 96, 98 of reciprocating driver 59 and the dosage adjuster 61 are configured to provide non-slip driving engagement when dosage adjuster 61 is moved in the direction of arrow 52 but for releasable engagement when dosage adjuster 61 is moved in the direction of arrow 64 once a sufficient separating force is applied between the reciprocating driver 59 and dosage adjuster 61.

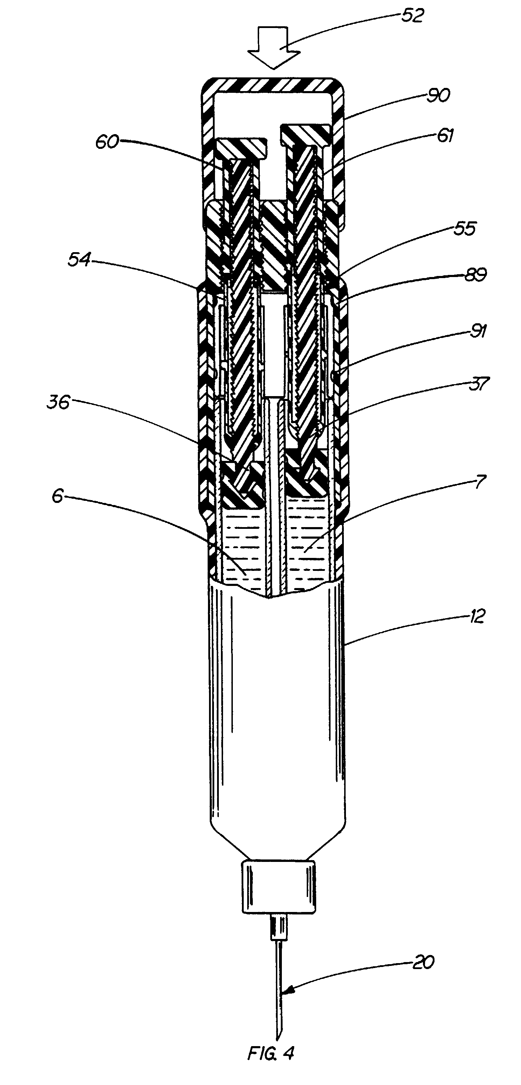

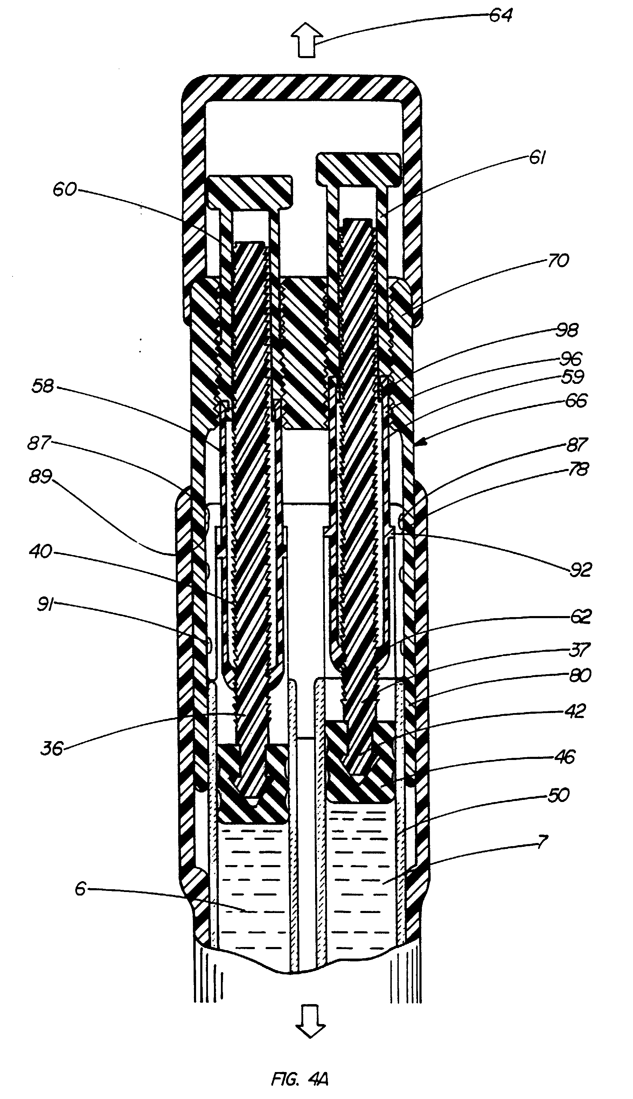

Fig. 4 shows dispenser 2 with cap 90 replaced and after reciprocating drive assembly 10 has been moved in the direction of arrow 52 during a delivery stroke from the pre-delivery condition of Fig. 3 to post-delivery condition of Fig. 4. Although not shown, rib 82 is in the position shown in Fig. 1 at the lower end of slot 84, rib 82 and slot 84 defining the limits of movement of drive assembly 10. Also, by comparing the positions of pistons 46 of cartridges 6, 7 it can be seen that a greater amount of the contents of cartridge 6 has been expulsed than of cartridge 7. This is due to the extra distance dosage adjuster 61 must travel before ends 96, 98 of reciprocating driver 59 and dosage adjuster 61 meet as compared with the corresponding ends of driver 58 and adjuster 60. The use of check valves 30 keep the contents of one cartridge 6, 7 from moving into the interior of the other cartridge 7, 6.

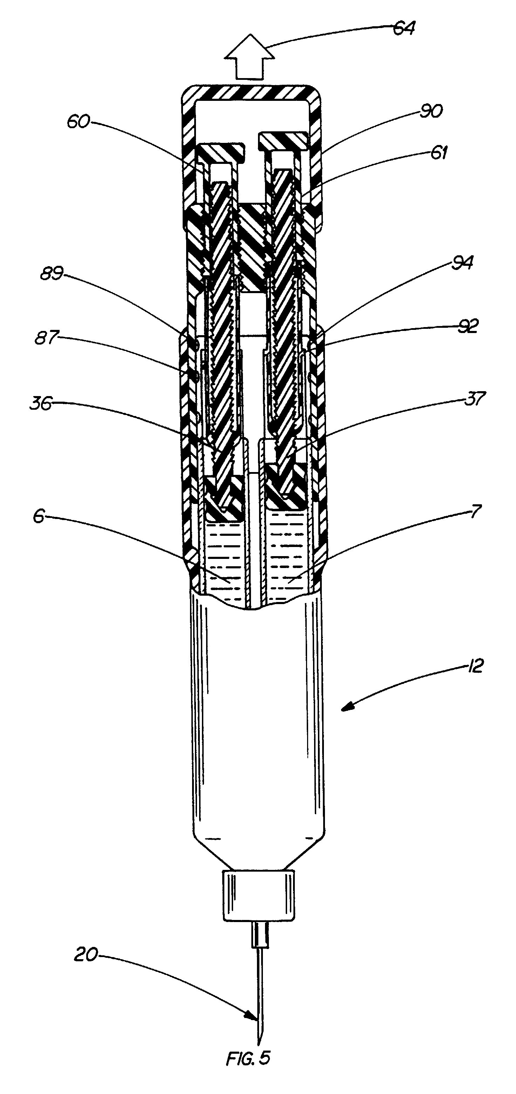

Fig. 5 illustrates variable proportion dispenser 2 with the reciprocating driver assembly 10 moved to an intermediate position between the post-delivery position of Fig. 4 by pulling in the direction of arrow 64. At this point, rib 82, not shown in Fig. 5, is mid-way along slot 84 but collar 92 of reciprocating driver 59 has engaged driver stop 94 while collar 92 of reciprocating 58 has not. Further movement of drive assembly 10 in the direction of arrow 64 will cause ends 96, 98 of one-way drive device 55 to separate as in Fig. 3. The engagement and separation of ends 96, 98 is facilitated by slits 99 in drivers 58, 59 which permit ends 96 to dilate.

To use dispenser 2, a new needle assembly 20 is generally mounted to tip 14 of housing 12. Cap 90 is removed is removed and dosage adjusters 60, 61 are each rotated according to the amount and proportions of the contents of cartridges 6, 7 to be injected per cycle. That is, for maximum dosage, the dosage adjusters 60, 61 are kept fully engaged within threaded holes 68 to minimize the distance between ends 96, 98. Numerical indicia 100, as suggested in Fig. 1, can be used on dosage adjusters 60, 61 to permit the appropriate injection volume and proportions to be chosen. Cap 90 is then replaced onto end 70 of sliding body 66, sliding body 66 is then moved in the direction of arrow 64 so that rib 82 moves from the position of Fig. 1 at the lower end of slot 84 to the upper end of slot 84. Doing so causes stem engaging ends 62 of reciprocating drivers 58, 59 to slide over drive stems 36, 37 so that pistons 46 do not move during this return stroke. (Friction between pistons 46 and cartridges 6, 7 is sufficient to keep drive stems 36, 37 in place during the return stroke.) Sliding body 66 is then driven downwardly in a delivery stroke in the direction of arrow 52 by pressing on cap 90. The contents of cartridges 6, 7 begin to be expulsed through associated spike tips 26, 27, check valves 30, bore 16 and needle assembly 20. In the configuration of Fig. 3, a greater proportion of the contents of cartridge 6 is expulsed through needle assembly 20 than of cartridge 7 because of the relative positions of dosage adjusters 60, 61. After use, needle assembly 20 can be capped or removed and a protective cap, not shown, can be mounted to tip 14 until the next use. To give another injection with the same volume and in the same proportions, one merely replaces needle assembly 20, if required, and moves sliding body 66 in a return stroke in the direction of arrow 64 and then in a delivery stroke in the direction of arrow 52, thus repeating the process.

The present invention has been described with reference to two cartridges 6, 7. The invention may be practiced with three or more cartridges as well. Also, other types of variable volume containers instead of pharmaceutical cartridges could also be used. For example, a collapsible bellows arrangement or a collapsible bag or sack could be used instead of the cartridges. Although the outer surfaces 40 of driver stems 36, 37 are serrated or toothed to provide a good ratcheting surface, the outer surfaces could be smooth as well by using other types of one-way drivers. The present invention is shown in an embodiment in which either component can be varied over a large range, preferably a range of 0% to 100%. If desired, adjustment devices could be provided that do not give such a wide range. For example, the adjustments could be such that the percentages of the components only range from 20% to 80% rather than 0% to 100%. In the preferred embodiment both the total volume of the dosage and the proportions are adjusted using dosage adjusters 60, 61. The total volume dispensed could also be adjusted by adjusting the effective length of slot 84. In addition, one of the components could be non-adjustable so that all adjustment in proportion would be through the reciprocating drive assembly for the other component; this might be useful when a separate means for adjusting the total volume dispensed is used, such as adjusting the effective length of slot 84. Dosage adjusters 60, 61 could also be coupled to one another through different sized gears. For example, dosage adjuster 60 could have 10-tooth gear while dosage adjuster 61 could have a 6-tooth gear so that every revolution of dosage adjuster 60 would cause dosage adjuster 61 to rotate one and two-third times. This could be useful if the ratio of the components is known and only the total volume is to be changed. Of course, different sets of gears for different ratios could be provided.

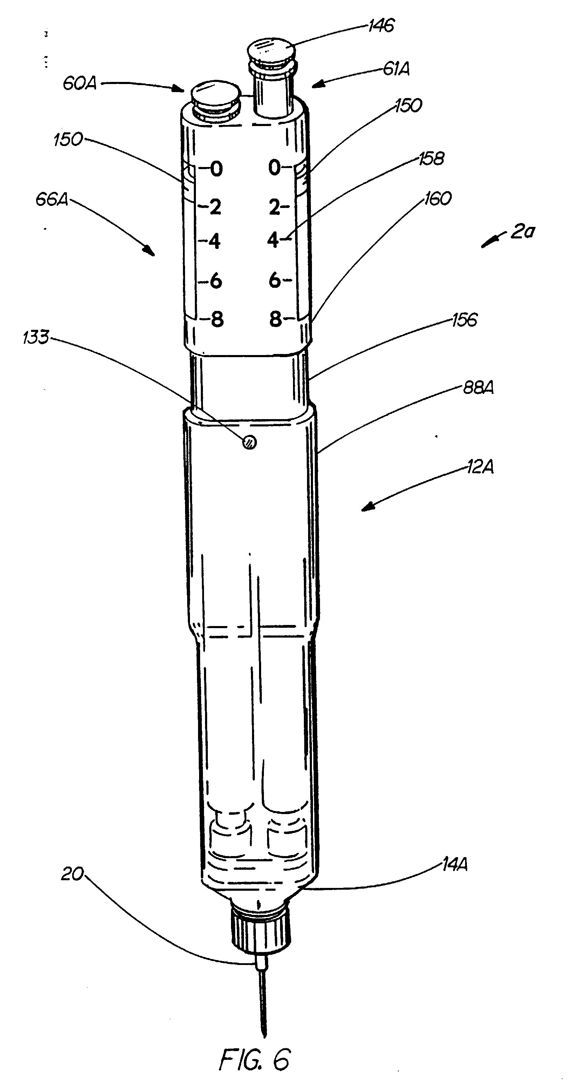

Figs. 6-8B illustrate an alternative embodiment of the invention shown in Figs. 1-5. Dispenser 2a is similar to dispenser 2 with corresponding reference numerals referring to corresponding parts; therefore parts which are identical will not be described separately. The primary differences between dispensers 2, 2a relate to the construction of dose adjustors 60a, 61a, sliding body 66a and check valve 30a.

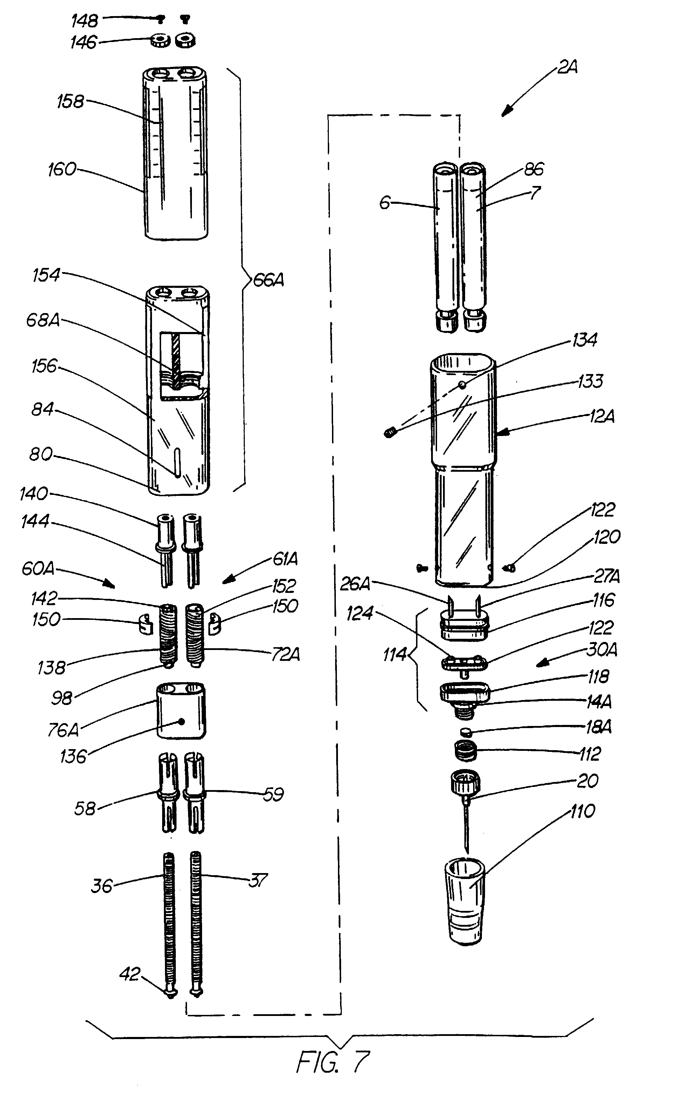

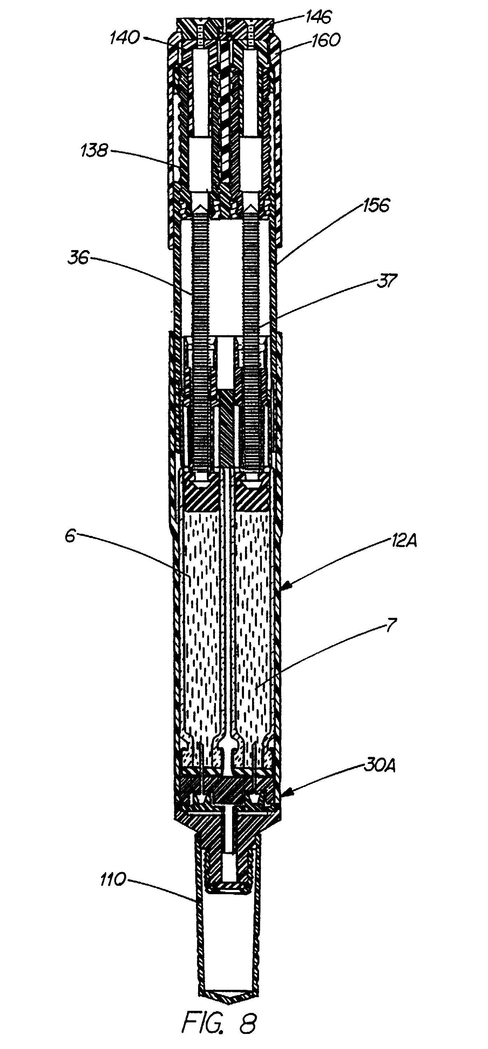

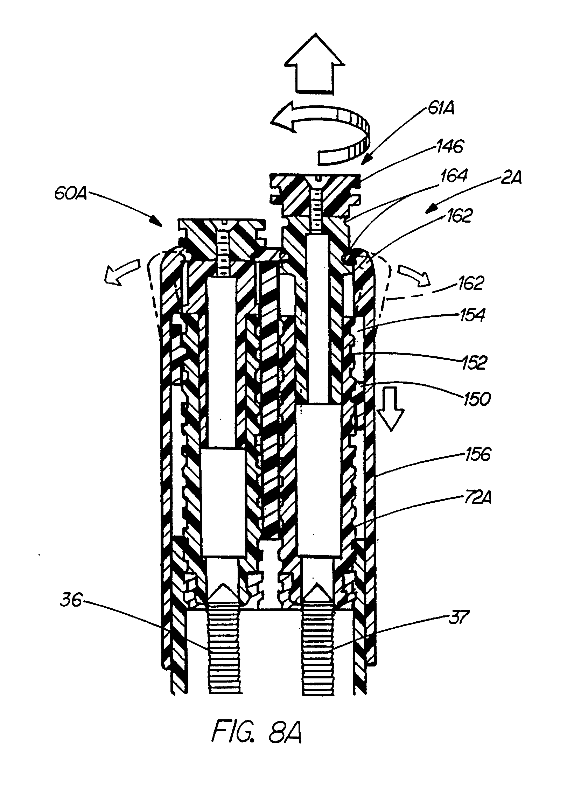

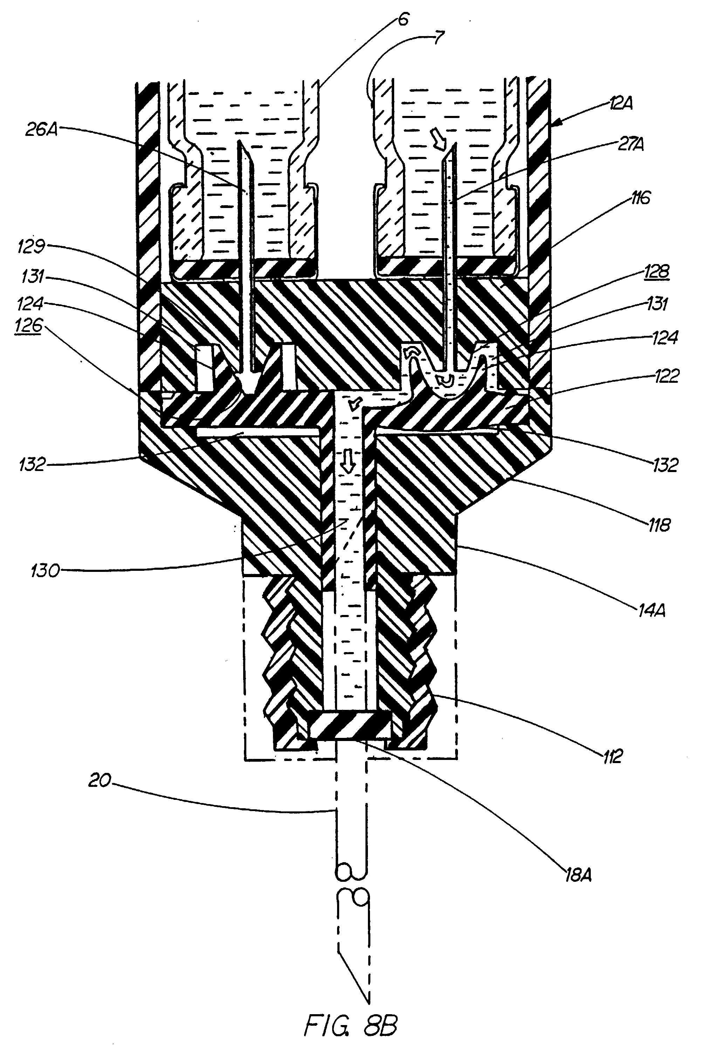

Referring the reader primarily to Fig. 7, a needle sheath 110 is used to cover needle assembly 20 prior to use for safety. Septum 18a, see Figs. 8 and 8B, is kept in place on tip 18a by a threaded keeper 112. Check valves 30a are formed differently from check valves 30. A combined check valve and spike assembly 114 includes a spike adapter 116, a manifold 118 and a check valve body 122. Manifold 118 carries tip 14a and is secured to an end 120 of housing 12a, such as through the use of an adhesive. Spike adaptor 116 is mounted within the interior of housing 12a at end 120 of housing 12a and is secured thereto by the use of screws 123 as shown in Fig. 7. Check valve body 122, made from butyl rubber, is positioned between spike adaptor 116 and manifold 118, both corresponding parts; therefore parts which are identical will not be described separately. The primary differences between dispensers 2, 2a relate to the construction of dose adjustors 60a, 61a, sliding body 66a and check valve 30a.

Referring the reader primarily to Fig. 7, a needle sheath 110 is used to cover needle assembly 20 prior to use for safety. Septum 18a, see Figs. 8 and 8B, is kept in place on tip 14a by a threaded keeper 112. Check valves 30a are formed differently from check valves 30. A combined check valve and spike assembly 114 includes a spike adapter 116, a manifold 118 and a check valve body 122. Manifold 118 carries tip 14a and is secured to an end 120 of housing 12a, such as through the use of an adhesive. Spike adaptor 116 is mounted within the interior of housing 12a at end 120 of housing 12a and is secured thereto by the use of screws 123 as shown in Fig. 7. Check valve body 122, made from butyl rubber, is positioned between spike adaptor 116 and manifold 118, both made of a hard plastic, such as polycarbonate. Check valve body 122 includes a pair of cup-shaped members 124 each having an internal conical surface 126 positioned to engage an external conical surface 128 formed by the outside of each of two projections 129. Projections 129 are hollow, as shown in Fig. 8A, and are positioned for fluid communication with the interior of hollow spikes 26a, 27a. Together check valve body 122, spike adaptor 116 and manifold 118 combine to create check valves 30a.

Normally, as shown in the left-hand side of Fig. 8B, check valves 30a are closed preventing fluid flow from a common pathway 130, which fluidly connects to the interior of needle cannula 20, to the interior of cartridges 6, 7. However, upon pressurization of the interior of one of the cartridges, such as cartridge 7 in Fig. 8B, the corresponding check valve 30a is opened as illustrated by the deformation of check valve body 122 and the arrows indicating fluid flow in Fig. 8B. To permit this deformation, assembly 114 provides an annular gap 131 surrounding cup-shaped members 124 and a further gap 132 in the space between that portion of check valve body 122 adjacent cup-shaped members 124 and manifold 118. The deformation of holes 68a in sliding body 66a for the same reasons and in the same manner as external threads 72 engage threaded holes 68 in the embodiment of Fig. 2. Threaded portion 138 has an oblong bore 142 sized to accept a similarly shaped oblong extension 144 of extension 140. The interface between bore 142 and extension 144 permits the free telescoping movement of extension 144 within bore 142 but causes rotary motion applied to extension 140 to rotate threaded portion 138.

As shown in Fig. 6, dose adjustor 60a also includes a dose control knob 146 which is secured to the end of extension 140 by a screw 148. Knob 146 can be grasped by a user and pulled away from sliding body 66a. Doing so permits the user to easily and independently rotate either dose adjustor 60a, 61a as desired without inadvertently rotating the other dose adjustor. For example, in Figs. 6 and 8A, dose knob 146 of dose adjustor 61a has been pulled away from sliding body 66a to permit the free rotation of the dose adjustor.

Rotation of dose knob 146, in addition to moving threaded portion 138 within sliding body 66a through the engagement of threads 72a and 68a, also causes the movement of a dose indicator 150. Dose indicator 150 engages left-hand threads 152 formed at the end of threaded portion 138 opposite right-hand threads 72a. Dose indicator 150 rides within a cutout 154 formed in the inner part 156 of sliding body 66a. Sliding body 66a also includes a transparent outer part 160, which carries indicator markings 158, mounted over and secured to inner part 156, such as with an adhesive. Cutout 154 keeps dose indicator 150 from moving in a rotary direction while allowing dose indicator 150 to be moved axially. In the preferred embodiment the pitch for threads 72a and 152 is the same. Accordingly, rotating dose knob 146 one complete revolution causes threaded portion 138 to move axially within sliding body 66a one pitch length of threads 72a. However, dose indicator 150, which engages left-hand threads 152 also moves one pitch length along threads 152. Thus, rotating dose knob 146 in a clockwise direction as indicated in Fig. 8A drives threaded portion 138 downwardly in the figure thus carrying dose indicator 150 with it. In addition to this movement, dose indicator 150 is moved one pitch length due to engagement of threads 72a and 68a, also causes the movement of a dose indicator 150. Dose indicator 150 engages left-hand threads 152 formed at the end of threaded portion 138 opposite right-hand threads 72a. Dose indicator 150 rides within a cutout 154 formed in the inner part 156 of sliding body 66a. Sliding body 66a also includes a transparent outer part 160, which carries indicator markings 158, mounted over and secured to inner part 156, such as with an adhesive. Cutout 154 keeps dose indicator 150 from moving in a rotary direction while allowing dose indicator 150 to be moved axially. In the preferred embodiment the pitch for threads 72a and 152 is the same. Accordingly, rotating dose knob 146 one complete revolution causes threaded portion 138 to move axially within sliding body 66a one pitch length of threads 72a. However, dose indicator 150, which engages left-hand threads 152 also moves one pitch length along threads 152. Thus, rotating dose knob 146 in a clockwise direction as indicated in Fig. 8A drives threaded portion 138 downwardly in the figure thus carrying dose indicator 150 with it. In addition to this movement, dose indicator 150 is moved one pitch length due to its engagement of the left-hand threads 152. This causes dose indicator 150 to move twice the distance traveled by threaded portion 138 and thus twice the distance travelled by drive stem 37. This magnifies the distance between indicator markings 158 by a two to one margin.

Fig. 8A illustrates dispenser 2a after dose knob 146 of dose adjustor 61a has been pulled outwardly to its operating position. Outer part 160 has a pair of spring fingers 162 formed at its upper end which engage grooves 164 formed in extensions 140. Spring fingers 162 and grooves 164 create detents which provide a positive indication to the user when dose adjustors 60a, 61a are in the retracted or extended positions. It should be noted that the axial movement of dose knob 146 together with extension 140 does not affect the operation of the unit; it only permits the appropriate dose adjustor to be rotated without affecting the rotary position, and thus the dose associated with the rotary position, of the other dose adjustor. If desired, the detents could be made so that the outermost groove 164 includes a series of notches while the innermost groove 164 is smooth so that, with reference to Fig. 8A, rotary motion of dose adjustor 61a is relatively unrestricted while rotary motion of dose adjustor 60a is substantially hindered or prevented.

Further modifications and variations can be made to the disclosed embodiments without departing from the subject of the invention as defined in the following claims.

## Claims

1. A variable proportion dispenser (2; 2A) comprising:
a housing (12; 12A);
first and second variable volume containers (6, 7) mounted to the housing (12; 12A) and including first and second exits and first and second movable elements (46) by which the contents of the first and second containers (6, 7) can be forced through said first and second exits as the first and second movable elements (46) are moved from first and second starting positions towards first and second ending positions; and
first and second drive stems (36, 37) drivingly coupled to the first and second movable elements (46);
characterized in that said variable proportion dispenser (2; 2A) further comprises:
reciprocal drive means (10) acting on said first and second drive stems (36, 37) for driving the first and second movable elements (46) from the first and second starting positions towards the first and second ending positions in a cyclic manner;
said reciprocal drive means (10) including:
first and second one-way drive devices (54, 55) for driving the first and second drive stems (36, 37) in a delivery direction during each cycle of said reciprocal drive means (10); each of said one-way drive devices (54, 55) comprising a reciprocating driver (58, 59) for driving the respective drive stem (36, 37) towards the respective container (6, 7) in the delivery direction and being movable relative to the drive stem (36, 37) in the reverse direction; and at least one of said one-way drive devices (54, 55) comprising means (60, 61; 60A, 61A) for adjusting the distance the reciprocating driver (58, 59) and the drive stem (36, 37) associated therewith travel in the delivery direction relative to the distance the reciprocating driver (58, 59) of the other one-way drive device (54, 55) and the other drive stem (36, 37) associated therewith travel in this delivery direction during each cycle of the reciprocal drive means (10).

2. The dispenser of claim 1 wherein said reciprocal drive means (10) includes first and second one-way drive devices (54, 55) for driving the first and second drive stems (36, 37), said first and second one-way drive devices (54, 55) comprising a first and a second reciprocating driver (58, 59), respectively, and said adjusting means (60, 61; 60A, 61A) is provided for adjusting the relative distances the first and second reciprocating drivers (58, 59) and the first and second drive stems (36, 37) associated therewith travel in the delivery direction during each cycle of the reciprocal drive means (10) so that the amounts and proportions of the contents of the first and second containers (6, 7) forced through the first and second exits during one or more cycles of the reciprocal drive means (10) can be optionally selected while said amounts and proportions remain the same unless said relative distances are changed.

3. The dispenser of claim 2 wherein the housing (12; 12A) is a clear plastic housing.

4. The dispenser of claim 2 wherein the variable volume containers include cartridges (6, 7) configured to contain pharmaceuticals.

5. The dispenser of claim 2 wherein said adjusting means (60, 61; 60A, 61A) are provided for individually adjusting the relative distance each of said first and second reciprocating drivers (58, 59) and said first and second drive stems (36, 37) associated therewith travel in the delivery direction during each cycle of the reciprocal drive means (10).

6. The dispenser of claim 2 wherein said adjusting means includes a rotatable dose control element (60, 61; 60A, 61A) for each of said first and second reciprocating drivers (58, 59) by which said amounts and proportions of the contents of the respective first and second containers (6, 7) forced through the first and second exits can be optionally selected.

7. The dispenser of claim 6 wherein each rotatable dose control element (60A, 61A) is an axially telescoping element to permit the user to easily rotatably manipulate one said dose control element (60A, 61A) without substantial interference from another said dose control element (60A, 61A).

8. The dispenser of claim 2 wherein the reciprocal drive means (10) includes means for independently adjusting the proportion of the contents of the first an second containers (6, 7) forced through the first and second exits during each cycle of the reciprocal drive means (10).

9. The dispenser of claim 2 wherein the first and second drive stems (36, 37) have serrated outer surfaces (40).

10. The dispenser of claim 2 further comprising means (150, 158) for indicating the amounts of the contents of each of the first and second containers (6, 7) which are to be forced through the first and second exits during the one or more cycles of the reciprocal drive means (10).

11. The dispenser of claim 10 wherein the indicating means (150, 158) includes first and second visual displays.

12. The dispenser of claim 11 wherein the housing (12; 12A) has first and second sides facing in opposite directions, said first and second displays located at the first and second sides, respectively.

13. The dispenser of claim 10 wherein the indicating means (150, 152, 158) includes:
first and second axially movable indicators (150) coupled to the first and second movable elements (46); and
means (152) for magnifying the axial movement of the first and second indicators (150) over the corresponding axial movement of the first and second movable elements (46).

14. The dispenser of claim 1 wherein said reciprocal drive means (10) comprises a sliding body (66; 66A) slidably mounted to the housing (12; 12A) for movement in delivery and reverse directions; a first reciprocating driver (58) is carried by the sliding body (66; 66A), the first reciprocating driver (58) and the first drive stem (36) are configured so that the first reciprocating driver (58) can move the first drive stem (36) towards the first variable volume container (6) only if the sliding body (66; 66A) is moving in the delivery direction; said adjusting means (60, 61; 60A, 61A) moves the first reciprocating driver (58) and the first drive stem (36) towards the first variable volume container (6) during the movement of the sliding body (66; 66A) in the delivery direction; and the second one-way drive (55) device is carried by the sliding body (66; 66A) configured to drive the second drive (37) stem towards the second variable volume container (7) when the sliding body (66; 66A) moves in the delivery direction.

15. The dispenser of claim 14 wherein the variable volume containers are cartridges (6, 7) configured to contain pharmaceuticals and the housing (12) is a clear plastic housing to provide visual access to the contents of the pharmaceutical cartridges (6, 7).

16. The dispenser of claim 14 wherein the first and second drive stems (36, 37) have serrated outer surfaces (40).

17. The dispenser of claim 14 wherein the one-way drive device (55) includes a second reciprocating driver (59), coupled to the second drive stem (37), configured so that the second reciprocating driver (59) can move the second drive stem (37) towards the second variable volume container (7) only if the sliding body (66, 66A) is moving in the delivery direction.

18. The dispenser of claim 17 wherein the one-way drive device (55) includes means (61; 61A) for adjusting the amount the second reciprocating driver (59) moves the second drive stem (37) towards the second variable volume container (7) during the movement of the sliding body (66; 66A) in the delivery direction.

19. The dispenser of claim 1 for dispensing multiple doses of first and second flowable materials at a chosen volumetric ratio, wherein said first and second variable volume containers (6,7) contain the first and second flowable materials; a sliding body (66; 66A) is reciprocally mounted to the housing (12; 12A) for movement parallel to an axis between first and second positions; a reciprocating driver (58) of the first one-way driver device (54) is operably coupled to the first drive stem (36) for driving the first stem (36) towards the first variable volume container (6); the second drive stem (37) is operably coupled to the sliding body (66; 66A) for movement parallel to the axis; said adjusting means (60, 61; 60A, 61A) comprises a dosage adjustor (60; 60A) adjustably mounted to the sliding body (66; 66A) to vary the axial position of the dosage adjustor (60; 60A) relative to the sliding body (66; 66A); the reciprocating driver (58) and the dosage adjustor (60; 60A) have mating portions (96, 98) configured to drivingly couple the dosage adjustor (60; 60A) to the reciprocating driver (58) when the sliding body (66; 66A) is moved from the first position to the second position in the delivery direction, and to permit the dosage adjustor (60; 60A) to be decoupled from the reciprocating driver (58) when the sliding body (66; 66A) is at an intermediate position between the first and second positions as the sliding body (66; 66A) is moved from the second position to the first position in the reverse direction; and the housing (12; 12A) and the reciprocating driver (58) include stop members (94, 92) positioned so that when the stop members (94, 92) are engaged, the movement of the reciprocating driver (58) is halted when the sliding body (66; 66A) reaches the intermediate position as the sliding body (66; 66A) moves from the second position to the first position in the reverse direction so to disengage the reciprocating driver (58) from the dosage adjustor (60; 60A).

## Patentansprüche

1. Abgabeeinrichtung (2; 2A) für veränderbare Verhältnisse umfassend:
ein Gehäuse (12; 12A);
erste und zweite Behälter (6, 7) mit veränderbarem Volumen, die an dem Gehäuse (12; 12A) befestigt sind und erste und zweite Ausgänge und erste und zweite bewegbare Elemente (46) umfassen, durch welche die Inhalte der ersten und zweiten Behälter (6, 7) durch die ersten und zweiten Ausgänge gedrängt werden können, wenn die ersten und zweiten bewegbaren Elemente (46) von ersten und zweiten Startpositionen zu ersten und zweiten Endpositionen bewegt werden; und
erste und zweite Antriebsstangen (36, 37), die treibend mit den ersten und zweiten bewegbaren Elementen (46) gekoppelt sind;
dadurch gekennzeichnet, daß die Abgabeeinrichtung (2; 2A) für veränderbare Verhältnisse ferner umfaßt:
eine hin- und herbewegbare Antriebseinrichtung (10), die auf die ersten und zweiten Antriebsstangen (36, 37) wirkt, um die ersten und zweiten bewegbaren Elemente (46) auf eine zyklische Art und Weise von den ersten und zweiten Startpositionen zu den ersten und zweiten Endpositionen anzutreiben;
wobei die hin- und herbewegbare Antriebseinrichtung (10) umfaßt:
erste und zweite Einwegantriebsvorrichtungen (54, 55) zum Antrieb der ersten und zweiten Antriebsstangen (36, 37) in einer Lieferrichtung während jedes Zyklus der hin- und herbewegbaren Antriebseinrichtung (10); wobei jede der Einwegantriebsvorrichtungen (54, 55) ein sich hin- und herbewegendes Antriebselement (58, 59) für den Antrieb der jeweiligen Antriebsstange (36, 37) in Richtung des jeweiligen Behälters (6, 7) in der Lieferrichtung umfaßt und relativ zu der Antriebsstange (36, 37) in der Umkehrrichtung bewegbar ist; und wobei zumindest eine der Einwegantriebsvorrichtungen (54, 55) eine Einrichtung (60, 61; 60A, 61A) umfaßt, um den Abstand, den das sich hin- und herbewegende Antriebselement (58, 59) und die damit zugeordnete Antriebstange (36, 37) in der Lieferrichtung durchläuft, relativ zu dem Abstand einzustellen, den das sich hin- und herbewegende Antriebselement (58, 59) der anderen Einwegantriebsvorrichtung (54, 55) und der anderen damit zugeordneten Antriebsstange (36, 37) in dieser Lieferrichtung während jedes Zyklus der hin- und herbewegbaren Antriebseinrichtung (10) durchläuft.

2. Abgabeeinrichtung nach Anspruch 1, wobei die hin- und herbewegbare Antriebseinrichtung (10) erste und zweite Einwegantriebsvorrichtungen (54, 55) umfaßt, um die ersten und zweiten Antriebsstangen (36, 37) anzutreiben, wobei die ersten und zweiten Einwegantriebsvorrichtungen (54, 55) ein erstes bzw. ein zweites sich hin- und herbewegendes Antriebselement (58, 59) umfassen, und die Einstelleinrichtung (60, 61; 60A, 61A) zum Einstellen der relativen Abstände vorgesehen ist, welche die ersten und zweiten sich hin- und herbewegenden Antriebselemente (58, 59) und die ersten und zweiten damit zugeordneten Antriebsstangen (36, 37) in der Lieferrichtung während jedes Zyklus der hin- und herbewegbaren Antriebseinrichtung (10) durchlaufen, so daß die Mengen und Verhältnisse der Inhalte der ersten und zweiten Behälter (6, 7), die durch die ersten und zweiten Ausgänge während eines oder mehrerer Zyklen der hin- und herbewegbaren Antriebseinrichtung (10) gedrängt werden, fakultativ gewählt werden können, während die Mengen und Verhältnisse gleich bleiben, sofern die relativen Abstände nicht geändert werden.

3. Abgabeeinrichtung nach Anspruch 2, wobei das Gehäuse (12; 12A) ein durchsichtiges Kunststoffgehäuse ist.

4. Abgabeeinrichtung nach Anspruch 2, wobei die Behälter mit veränderbarem Volumen Patronen (6, 7) umfassen, die zur Aufnahme von pharmazeutischen Stoffen ausgeführt sind.

5. Abgabeeinrichtung nach Anspruch 2, wobei die Einstelleinrichtungen (60, 61; 60A, 61A) zum individuellen Einstellen des relativen Abstandes vorgesehen sind, den jedes der ersten und zweiten sich hin- und herbewegenden Antriebselemente (58, 59) und die damit zugeordneten ersten und zweiten Antriebsstangen (36, 37) in der Lieferrichtung während jedes Zyklus der hin- und herbewegbaren Antriebseinrichtung (10) durchlaufen.

6. Abgabeeinrichtung nach Anspruch 2, wobei die Einstelleinrichtung ein drehbares Dosissteuerelement (60, 61; 60A, 61A) für jedes der ersten und zweiten sich hin- und herbewegenden Antriebselemente (58, 59) umfaßt, durch welches die Mengen und Verhältnisse der Inhalte der jeweiligen ersten und zweiten Behälter (6, 7), die durch die ersten und zweiten Ausgänge gedrängt werden, fakultativ gewählt werden können.

7. Abgabeeinrichtung nach Anspruch 6, wobei jedes drehbare Dosissteuerelement (60A, 61A) ein axial ausfahrbares Element ist, um zu ermöglichen, daß der Anwender ein derartiges Dosissteuerelement (60A, 61A) ohne wesentliche Überlagerung mit dem anderen Dosissteuerelement (60A, 61A) leicht drehbar betätigen kann.

8. Abgabeeinrichtung nach Anspruch 2, wobei die hin- und herbewegbare Antriebseinrichtung (10) eine Einrichtung umfaßt, um den Prozentsatz der Inhalte der ersten und zweiten Behälter (6, 7) unabhängig einzustellen, die durch die ersten und zweiten Ausgänge während jedes Zyklus der hin- und herbewegbaren Antriebseinrichtung (10) gedrängt werden.

9. Abgabeeinrichtung nach Anspruch 2, wobei die ersten und zweiten Antriebsstangen (36, 37) eingekerbte Außenflächen (40) aufweisen.

10. Abgabeeinrichtung nach Anspruch 2, ferner umfassend eine Vorrichtung (150, 158) zur Anzeige der Mengen der Inhalte von jedem der ersten und zweiten Behälter (6, 7), die durch die ersten und zweiten Ausgänge während eines oder mehrerer Zyklen der hin- und herbewegbaren Antriebseinrichtung (10) gedrängt werden.

11. Abgabeeinrichtung nach Anspruch 10, wobei die Anzeigevorrichtung (150, 158) erste und zweite optische Anzeigen umfaßt.

12. Abgabeeinrichtung nach Anspruch 11, wobei das Gehäuse (12; 12A) erste und zweite Seiten aufweist, die in entgegengesetzte Richtungen weisen, wobei die ersten und zweiten Anzeigen an den ersten bzw. zweiten Seiten angeordnet sind.

13. Abgabeeinrichtung nach Anspruch 10, wobei die Anzeigevorrichtung (150, 152, 158) umfaßt:
erste und zweite axial bewegbare Anzeigeeinrichtungen (150), die mit den ersten und zweiten bewegbaren Elementen (46) gekoppelt sind; und
eine Einrichtung (152) zur Vergrößerung der Axialbewegung der ersten und zweiten Anzeigeeinrichtungen (150) über die entsprechende Axialbewegung der ersten und zweiten bewegbaren Elemente (46).

14. Abgabeeinrichtung nach Anspruch 1, wobei die hin- und herbewegbare Antriebseinrichtung (10) einen Verschiebekörper (66; 66A) umfaßt, der an dem Gehäuse (12; 12A) für eine Bewegung in den Liefer- und Umkehrrichtungen verschiebbar befestigt ist; ein erstes sich hin- und herbewegendens Antriebselement (58) von dem Verschiebekörper (66, 66A) getragen wird, das erste sich hin- und herbewegende Antriebselement (58) und die erste Antriebsstange (36) so ausgeführt sind, daß das erste sich hin- und herbewegende Antriebselement (58) die erste Antriebsstange (36) in Richtung des ersten Behälters (6) mit veränderbarem Volumen nur dann bewegen kann, wenn der Verschiebekörper (66; 66A) in die Lieferrichtung bewegt wird; die Einstelleinrichtung (60, 61; 60A, 61A) das erste sich hin- und herbewegende Antriebselement (58) und die erste Antriebsstange (36) in Richtung des ersten Behälters (6) mit veränderbarem Volumen während der Bewegung des Verschiebekörpers (66; 66A) in der Lieferrichtung bewegt; und die zweite Einwegantriebsvorrichtung (55) von dem Verschiebekörper (66; 66A) getragen wird und so ausgeführt ist, daß die zweite Antriebsstange (37) in Richtung des zweiten Behälters (7) mit veränderbarem Volumen getrieben wird, wenn der Verschiebekörper (66; 66A) in die Lieferrichtung bewegt wird.

15. Abgabeeinrichtung nach Anspruch 14, wobei die Behälter mit veränderbarem Volumen Patronen (6, 7) sind, die so ausgeführt sind, daß sie pharmazeutische Stoffe enthalten können, und das Gehäuse (12) ein durchsichtiges Kunststoffgehäuse ist, um einen Sichtkontakt zu den Inhalten der pharmazeutischen Patronen (6, 7) zu schaffen.

16. Abgabeeinrichtung nach Anspruch 14, wobei die ersten und zweiten Antriebsstangen (36, 37) eingekerbte Außenflächen (40) aufweisen.

17. Abgabeeinrichtung nach Anspruch 14, wobei die Einwegantriebsvorrichtung (55) ein zweites sich hin- und herbewegendes Antriebselement (59) umfaßt, das mit der zweiten Antriebsstange (37) gekoppelt ist und so ausgeführt ist, daß das zweite sich hin- und herbewegende Antriebselement (59) die zweite Antriebsstange (37) in Richtung des zweiten Behälters (7) mit veränderbarem Volumen nur dann bewegen kann, wenn der Verschiebekörper (66, 66A) in der Lieferrichtung bewegt wird.

18. Abgabeeinrichtung nach Anspruch 17, wobei die Einwegantriebsvorrichtung (55) eine Einrichtung (61; 61A) zum Einstellen des Ausmaßes umfaßt, in welchem das zweite sich hin- und herbewegende Antriebselement (59) die zweite Antriebsstange (37) in Richtung des zweiten Behälters (7) mit veränderbarem Volumen während der Bewegung des Verschiebekörpers (66; 66A) in der Lieferrichtung bewegt.

19. Abgabeeinrichtung nach Anspruch 1 zur Abgabe von mehrfachen Dosen eines ersten und zweiten fließbaren Materials bei einem gewählten volumetrischen Verhältnis, wobei die ersten und zweiten Behälter (6, 7) mit veränderbarem Volumen die ersten und zweiten fließbaren Materialien enthalten; ein Verschiebekörper (66; 66A) hin- und herbewegbar an dem Gehäuse (12; 12A) für eine Bewegung parallel zu einer Achse zwischen ersten und zweiten Positionen befestigt ist; ein sich hin- und herbewegendes Antriebselement (58) der ersten Einwegantriebsvorrichtung (54) wirksam mit der ersten Antriebsstange (36) gekoppelt ist, um die erste Stange (36) in Richtung des ersten Behälters (6) mit veränderbarem Volumen zu treiben; die zweite Antriebsstange (37) wirksam mit dem Verschiebekörper (66; 66A) für eine Bewegung parallel zu der Achse gekoppelt ist; die Einstelleinrichtung (60, 61; 60A, 61A) eine Dosiereinstelleinrichtung (60; 60A) umfaßt, die einstellbar an dem Verschiebekörper (66; 66A) befestigt ist, um die axiale Position der Dosiereinstelleinrichtung (60; 60A) relativ zu dem Verschiebekörper (66; 66A) zu ändern; das sich hin- und herbewegende Antriebselement (58) und die Dosiereinstelleinrichtung (60; 60A) zusammenpassende Abschnitte (96, 98) aufweisen, die so ausgeführt sind, daß die Dosiereinstelleinrichtung (60; 60A) treibend mit dem sich hin- und herbewegenden Antriebselement (58) gekoppelt ist, wenn der Verschiebekörper (66; 66A) von der ersten Position in die zweite Position in der Lieferrichtung bewegt wird, und um zuzulassen, daß die Dosiereinstelleinrichtung (60; 60A) von dem sich hin- und herbewegenden Antriebselement (58) entkoppelt werden kann, wenn der Verschiebekörper (66; 66A) an einer Zwischenposition zwischen den ersten und zweiten Positionen liegt, wenn der Verschiebekörper (66; 66A) von der zweiten Position zu der ersten Position in der Umkehrrichtung bewegt wird; und das Gehäuse (12; 12A) und das sich hin- und herbewegende Antriebselement (58) Stoppelemente (94, 92) umfassen, die so positioniert sind, daß, wenn die Stoppelemente (94, 92) in Eingriff stehen, die Bewegung des sich hin- und herbewegenden Antriebselementes (58) angehalten wird, wenn der Verschiebekörper (66; 66A) die Zwischenposition erreicht, wenn sich der Verschiebekörper (66; 66A) von der zweiten Position zu der ersten Position in der Umkehrrichtung bewegt, um so das sich hin- und herbewegende Antriebselement (58) von der Dosiereinstelleinrichtung (60; 60A) auszurücken.

## Revendications

1. Distributeur de proportions variables (2 ; 2A), comprenant :
un boîtier (12 ; 12 A) ;
un premier et un deuxième récipient à volume variable (6, 7) montés sur le boîtier (12 ; 12A) et comprenant une première et une deuxième sortie, et un premier et un deuxième élément mobile (46) au moyen desquels les contenus du premier et du deuxième récipient (6, 7) peuvent être forcés à travers ladite première et ladite deuxième sortie tandis que le premier et le deuxième élément mobile (46) sont déplacés depuis une première et une deuxième position de départ en direction d'une première et d'une deuxième position terminale ; et
une première et une deuxième tige d'entraînement (36, 37) accouplées en termes d'entraînement au premier et au deuxième élément mobile (46) ;
caractérisé en ce que ledit distributeur de proportions variables (2 ; 2A) comprend en outre :
des moyens d'entraînement en va-et-vient (10) agissant sur ladite première et ladite deuxième tige d'entraînement (36, 37) pour entraîner le premier et le deuxième élément mobile (46) depuis la première et la deuxième position de départ vers la première et la deuxième position terminale d'une manière cyclique ;
lesdits moyens d'entraînement en va-et-vient (10) comprenant :
un premier et un deuxième dispositif d'entraînement unidirectionnel (54, 55) pour entraîner la première et la deuxième tige d'entraînement (36, 37) dans une direction de distribution pendant chaque cycle desdits moyens d'entraînement en va-et-vient (10) ; chacun desdits dispositifs d'entraînement unidirectionnel (54, 55) comprenant un élément d'entraînement à va-et-vient (58, 59) pour entraîner la tige d'entraînement respective (36, 37) vers le récipient respectif (6, 7) dans la direction de distribution et étant mobile par rapport à la tige d'entraînement (36, 37) dans la direction inverse ; et l'un au moins desdits dispositifs d'entraînement unidirectionnel (54, 55) comprenant des moyens (60, 61 ; 60A, 61A) pour ajuster la distance sur laquelle l'élément d'entraînement à va-et-vient (58, 59) et la tige d'entraînement (36, 37) associée à celui-ci se déplacent dans la direction de distribution par rapport à la distance sur laquelle l'élément d'extrêmement à va-et-vient (58, 59) de l'autre dispositif d'entraînement unidirectionnel (54, 55) et l'autre tige d'entraînement (36, 37) associée à celui-ci se déplacent dans cette direction de distribution pendant chaque cycle des moyens d'entraînement à va-et-vient (10).

2. Distributeur selon la revendication 1, dans lequel lesdits moyens d'entraînement à va-et-vient (10) incluent un premier et un deuxième dispositif d'entraînement unidirectionnel (54, 55) pour entraîner la première et la deuxième tige d'entraînement (36, 37), ledit premier et ledit deuxième dispositif d'entraînement unidirectionnel (54, 55) comprenant un premier et deuxième élément d'entraînement à va-et-vient (58, 59), respectivement, et lesdits moyens d'ajustement (60, 61 ; 60A, 61A) sont prévus pour ajuster les distances relatives sur lesquelles le premier et le deuxième élément d'entraînement à va-et-vient (58, 59) et la première et la deuxième tige d'entraînement (36, 37) associées à ceux-ci se déplacent dans la direction de distribution pendant chaque cycle des moyens d'entraînement à va-et-vient (10), de sorte que les quantités et les proportions des contenus du premier et du deuxième récipient (6, 7) forcés à travers la première et la deuxième sortie pendant un ou plusieurs cycles des moyens d'entraînement à va-et-vient (10) peuvent être choisis en option cependant que lesdites quantités et lesdites proportions restent les mêmes à moins de changer lesdites distances relatives.

3. Distributeur selon la revendication 2, dans lequel le boîtier (12 ; 12A) est un boîtier en matière plastique claire.

4. Distributeur selon la revendication 2, dans lequel les récipients à volume variable incluent des cartouches (6, 7) configurées pour contenir des produits pharmaceutiques.

5. Distributeur selon la revendication 2, dans lequel lesdits moyens d'ajustement (60, 61 ; 60A, 61A) sont prévus pour ajuster individuellement la distance relative sur laquelle chacun desdits premier et deuxième éléments d'entraînement à va-et-vient (58, 59) et desdites première et deuxième tiges d'entraînement (36, 37) associées à ceux-ci se déplacent dans la direction de distribution pendant chaque cycle des moyens d'entraînement en va-et-vient (10).

6. Distributeur selon la revendication 2, dans lequel lesdits moyens d'ajustement incluent un élément de commande de dosage rotatif (60, 61 ; 60A, 61A) pour chacun desdits premier et deuxième éléments d'entraînement à va-et-vient (58, 59) au moyen desquels lesdites quantités et lesdites proportions des contenus du premier et du deuxième récipient respectif (6, 7) forcés à travers la première et la deuxième sortie peuvent être choisies en option.

7. Distributeur selon la revendication 6, dans lequel chaque élément de commande de dosage rotatif (60A, 61A) est un élément télescopique en direction axiale pour permettre à l'utilisateur de manipuler aisément en rotation l'un desdits éléments de commande de dosage (60A, 61A) sans une interférence sensible depuis un autre desdits éléments de commande de dosage (60A, 61A).

8. Distributeur selon la revendication 2, dans lequel les moyens d'entraînement en va-et-vient (10) incluent des moyens pour ajuster indépendamment la proportion des contenus du premier et du deuxième récipient (6, 7) forcés à travers la première et la deuxième sortie pendant chaque cycle des moyens d'entraînement en va-et-vient (10).

9. Distributeur selon la revendication 2, dans lequel la première et la deuxième tige d'entraînement (36, 37) possèdent des surfaces extérieures cannelées (40).

10. Distributeur selon la revendication 2, comprenant en outre des moyens (150, 158) pour indiquer les quantités des contenus de chacun parmi le premier et le deuxième récipient (6, 7) qu'il s'agit de forcer à travers la première et la deuxième sortie pendant un ou plusieurs cycles des moyens d'entraînement en va-et-vient (10).

11. Distributeur selon la revendication 10, dans lequel les moyens d'indication (150, 158) incluent un premier et deuxième affichage visuel.

12. Distributeur selon la revendication 11, dans lequel le boîtier (12 ; 12A) présente un premier et un deuxième côté tournés vers des directions opposées, ledit premier et ledit deuxième affichage étant situés respectivement au niveau du premier et du deuxième côté.

13. Distributeur selon la revendication 10, dans lequel les moyens d'indication (150, 152, 158) incluent :
un premier et un deuxième indicateur axialement mobiles (150) accouplés au premier et au deuxième élément mobile (46) ; et
des moyens (152) pour amplifier le mouvement axial du premier et du deuxième indicateur (150) par rapport au mouvement axial correspondant du premier et du deuxième élément mobile (46).

14. Distributeur selon la revendication 1, dans lequel lesdits moyens d'entraînement en va-et-vient (10) comprennent un corps coulissant (66 ; 66A) monté en coulissement dans le boîtier (12 ; 12A) en vue d'un mouvement dans la direction de distribution et dans la direction inverse ; un premier élément d'entraînement à va-et-vient (58) étant porté par le corps coulissant (66 ; 66A), le premier élément d'entraînement à va-et-vient (58) et la première tige d'entraînement (36) étant configurés de telle façon que le premier élément d'entraînement à va-et-vient (58) peut déplacer la première tige d'entraînement (36) vers le premier récipient à volume variable (6) uniquement si le corps coulissant (66 ; 66A) se déplace dans la direction de distribution; lesdits moyens d'ajustement (60, 61 ; 60A, 61A) déplaçant le premier élément d'entraînement à va-et-vient (58) et la première tige d'entraînement (36) vers le premier récipient à volume variable (6) pendant le mouvement du corps coulissant (66 ; 66A) dans la direction de distribution ; et le deuxième dispositif d'entraînement unidirectionnel (55) étant porté par le corps coulissant (66 ; 66A), configuré pour entraîner la deuxième tige d'entraînement (37) vers le deuxième récipient à volume variable (7) lorsque le corps coulissant (66 ; 66A) se déplace dans la direction de distribution.

15. Distributeur selon la revendication 14, dans lequel les récipients à volume variable sont des cartouches (6, 7) configurées pour contenir des produits pharmaceutiques, et le boîtier (12) est un boîtier en matière plastique claire pour fournir un accès visuel aux contenus des cartouches pharmaceutiques (6, 7).

16. Distributeur selon la revendication 14, dans lequel la première et la deuxième tige entraînement (36, 37) présentent des surfaces extérieures cannelées (40).

17. Distributeur selon la revendication 14, dans lequel le dispositif d'entraînement unidirectionnel (55) inclut un deuxième élément d'entraînement à va-et-vient (59), accouplé à la deuxième tige d'entraînement (37), et configuré de telle façon que le deuxième élément d'entraînement à va-et-vient (59) peut déplacer la deuxième tige d'entraînement (37) vers le deuxième récipient à volume variable (7) uniquement si le corps coulissant (66, 66A) se déplace dans la direction de distribution.

18. Distributeur selon la revendication 17, dans lequel le dispositif d'entraînement unidirectionnel (55) inclut des moyens (61 ; 61A) pour ajuster la distance sur laquelle le deuxième élément d'entraînement à va-et-vient (59) déplace la deuxième tige d'entraînement (37) vers le deuxième récipient à volume variable (7) pendant le mouvement du corps coulissant (66 ; 66A) dans la direction de distribution.

19. Distributeur selon la revendication 1, destiné à distribuer des doses multiples d'un premier et d'un deuxième matériau capables de s'écouler, sous un rapport volumétrique choisi, dans lequel ledit premier et ledit deuxième récipient à volume variable (6, 7) contiennent le premier et le deuxième matériau capables de s'écouler ; un corps coulissant (66 ; 66A) étant monté en va-et-vient sur le boîtier (12 ; 12A) en vue d'un mouvement parallèle à un axe entre une première et une deuxième position ; un élément d'entraînement à va-et-vient (58) du premier dispositif d'entraînement à va-et-vient (54) étant fonctionnellement accouplé à la première tige d'entraînement (36) pour entraîner la première tige (36) vers le premier récipient à volume variable (6) ; la deuxième tige d'entraînement (37) étant fonctionnellement accouplée au corps coulissant (66 ; 66A) en vue d'un mouvement parallèle à l'axe ; lesdits moyens d'ajustement (60, 61 ; 60A, 61A) comprenant un ajusteur de dosage (60 ; 60A) monté de façon réglable sur le corps coulissant (66 ; 66A) pour faire varier la position axiale de l'ajusteur de dosage (60 ; 60A) par rapport au corps coulissant (66 ; 66A) ; l'élément d'entraînement à va-et-vient (58) et l'ajusteur de dosage (60 ; 60A) possédant des parties appariées (96, 98) configurées de manière à accoupler en entraînement l'ajusteur de dosage (60 ; 60A) à l'élément d'entraînement à va-et-vient (58) lorsque le corps coulissant (66 ; 66A) est déplacé depuis la première position jusqu'à la deuxième position dans la direction de distribution, et pour permettre à l'ajusteur de dosage (60 ; 60A) d'être découplé depuis l'élément d'entraînement à va-et-vient (58) lorsque le corps coulissant (66 ; 66A) se trouve à une position intermédiaire entre la première et la deuxième position tandis que le corps coulissant (66 ; 66A) est déplacé depuis la deuxième position jusqu'à la première position dans la direction inverse ; et le boîtier (12 ; 12A) et l'élément d'entraînement à va-et-vient (58) incluent des éléments d'arrêt (94, 92) positionnés de sorte que lorsque les éléments d'arrêt (94, 92) sont engagés, le mouvement de l'élément d'entraînement à va-et-vient (58) est arrêté lorsque le corps coulissant (66 ; 66A) se déplace depuis la deuxième position jusqu'à la première position dans la direction inverse, pour dégager ainsi l'élément d'entraînement à va-et-vient (58) depuis l'ajusteur de dosage (60 ; 60A).
